# EUROPÄISCHE PATENTSCHRIFT

(11) **EP 2 760 515 B1**
(45) Veröffentlichungstag und Bekanntmachung des Hinweises auf die Patenterteilung: **25.10.2017**
(21) Anmeldenummer: 12770079.7
(22) Anmeldetag: 27.09.2012
(51) Int. Cl.: A61M 16/00, A61M 16/10, A61M 16/16, F21V 33/00

(54) **BELEUCHTUNGSVORRICHTUNG FÜR ATEMLUFTBEFEUCHTER UND ATEMLUFTBEFEUCHTER**
LIGHTING DEVICE FOR A RESPIRATORY HUMIDIFIER AND RESPIRATORY HUMIDIFIER
DISPOSITIF D'ÉCLAIRAGE POUR HUMIDIFICATEUR D'AIR INHALÉ ET HUMIDIFICATEUR D'AIR INHALÉ

(30) Priorität: 01.10.2011 DE 102011054135
(43) Veröffentlichungstag der Anmeldung: 06.08.2014
(73) Patentinhaber: Hamilton Medical AG, 7402 Bonaduz (CH)
(72) Erfinder: BÜCHI, Rudolf, CH-7000 Chur (CH)
(74) Vertreter: Caspary, Karsten
(86) Internationale Anmeldenummer: PCT/EP2012/069128
(87) Internationale Veröffentlichungsnummer: WO 2013/045578

(56) Entgegenhaltungen:
- EP-A2- 0 376 584
- WO-A1-2010/031126
- WO-A2-2012/065999
- US-A- 4 028 444

## Beschreibung

Die vorliegende Erfindung betrifft einen Atemluftbefeuchter mit Beleuchtungsvorrichtung beim Beatmen von Patienten mit Atemgas.

Bei der maschinellen Beatmung von Patienten, die beispielsweise auf einer Intensivstation liegen, wird der zu beatmende Patient mit Hilfe eines Beatmungsschlauchsystems pneumatisch mit dem Beatmungsgerät verbunden. Da das Atemgas, das dem Patienten zugeführt wird, hinsichtlich Temperatur und Feuchtigkeit den physiologischen Erfordernissen des Patienten angepasst werden muss, wird ein Atemluftbefeuchter in dem Einatem- oder Inspirationsschlauch angeordnet, der das Atemgas anwärmt und anfeuchtet. Der Atemluftbefeuchter weist einen mit destilliertem Wasser gefüllten Flüssigkeitsbehälter auf, durch den das Einatemgas geleitet und mit Feuchtigkeit angereichert wird.

Die Aufheizung der Flüssigkeit in dem Flüssigkeitsbehälter wird normalerweise über eine Heizplatte im unteren Abschnitt des Gehäuses des Atemluftbefeuchters bewirkt, wobei die Wärmeübertragung thermisch von der Heizplatte auf den wärmeleitfähigen Boden des Flüssigkeitsbehälters erfolgt. Die Temperatur des Atemgases wird beispielsweise beim Einströmen und beim Ausströmen durch geeignete Sensoren gemessen.

Damit der Flüssigkeitsbehälter nicht trocken läuft, wird der Füllstand im Flüssigkeitsbehälter überwacht. Bei Unterschreiten eines Minimalwertes wird beispielsweise über ein Schwimmerventil Flüssigkeit aus einem externen Reservoir zugeführt. Ein Signal an den Benutzer, dass dieser auf das Nachfüllen von Flüssigkeit und gegebenenfalls auf das Erneuern des Reservoirs aufmerksam gemacht wird, wird dabei nicht ausgegeben.

Es ist ebenfalls bekannt, Atemluftbefeuchter mit Benutzerschnittstellen auszustatten, die Anzeige- und Bedienelemente aufweisen. Eine Steuereinrichtung regelt bzw. steuert die Funktion des Atemluftbefeuchters und gibt gegebenenfalls ein optisches oder akustisches Signal aus, wenn ein alarmwürdiger Funktionszustand vorliegt. Durch die kleinen Abmessungen sind die Möglichkeiten für ein optisch deutliches Alarmsignal begrenzt, beispielsweise auf kleinflächige LEDs, die die Anzeigeelemente bilden.

Eine weitere Anforderung in einem Atemluftbefeuchter ist, dass der Flüssigkeitsbehälter als medizinischer Einmal-/Wegwerfartikel besonders einfach und kostengünstig herstellbar sein muss. Eine Beleuchtungskonstruktion darf daher nicht komplex sein und sollte möglichst wenige Bauelemente umfassen sowie mit vertretbarem Aufwand herstellbar sein.

Das Dokument WO 2010/031126 A1 offenbart einen Atemluftbefeuchter gemäß dem Oberbegriff des Anspruchs 1. Es ist daher die Aufgabe der vorliegenden Erfindung, eine Beleuchtungsvorrichtung für einen Atemluftbefeuchter bereitzustellen, die einen Alarmzustand optisch sehr deutlich in möglichst viele Richtungen signalisieren kann und dabei mit einem einfachen Aufbau kostengünstig realisiert werden kann.

Diese Aufgabe wird durch die Merkmale des Anspruchs 1 gelöst. Vorteilhafte Ausgestaltungen und Ausführungsformen sind in den Unteransprüchen beschrieben.

Erfindungsgemäß wird ein Atemluftbefeuchter mit Beleuchtungsvorrichtung bereitgestellt, der einen Flüssigkeitsbehälter mit einer Bodenplatte und ein Gehäuse mit einer Heizplatte aufweist, wobei zur Beheizung der Flüssigkeit im Flüssigkeitsbehälter die Heizplatte erwärmbar und die Bodenplatte in Kontakt mit der Heizplatte bringbar ist, wobei das Gehäuse ein Leuchtmittel bzw. eine Lichtquelle und einen Lichtaustrittsabschnitt aufweist und der Flüssigkeitsbehälter einen Lichteintrittsabschnitt aufweist, wobei das Licht ausgehend vom Leuchtmittel in einer Leuchtrichtung durch den Lichtaustrittsabschnitt und den Lichteintrittsabschnitt in den Flüssigkeitsbehälter eintritt, um einen Alarmzustand des Atemluftbefeuchter optisch für einen Benutzer zu signalisieren, dadurch gekennzeichnet, dass der Lichtaustrittsabschnitt als ein transparenter Wandabschnitt des Gehäuses ausgebildet ist, die Leuchtrichtung im Wesentlichen auf die Bodenplatte gerichtet ist, und dass die Wandflächen des Flüssigkeitsbehälters zumindest abschnittsweise transparent sind. Durch das Hineinleuchten von innerhalb des Gehäuses in den Flüssigkeitsbehälter, d. h. auf die Flüssigkeitsoberfläche, ergibt sich ein relativ großer beleuchteter Bereich, der durch die Reflexion des Lichtes auf der Flüssigkeitsoberfläche noch weiter vergrößert wird. Zudem kommt der Flüssigkeitsbehälter völlig ohne aktive elektrische Bauelemente aus. Erfindungsgemäß ist die Leuchtrichtung im Wesentlichen auf die Bodenplatte gerichtet und die Wandflächen des Flüssigkeitsbehälters sind zumindest abschnittsweise transparent. Die Leuchtrichtung auf die Bodenplatte gewährleistet, dass das Licht im Flüssigkeitsbehälter auf die reflektierende Oberfläche der Flüssigkeit fällt und damit den Signalisierungseffekt der Beleuchtung verstärkt. Wenn die Wandflächen des Flüssigkeitsbehälters zumindest abschnittsweise transparent sind, dann ist die Beleuchtung des Flüssigkeitsbehälters von außerhalb noch besser sichtbar.

Mit weiterem Vorteil weisen der Lichtaustrittsabschnitt und der Lichteintrittsabschnitt zueinander passend geformte Oberflächen auf, die mit einem Luftspalt dazwischen aneinander angrenzen.

Besonders bevorzugt ist, wenn der Lichtaustrittsabschnitt und der Lichteintrittsabschnitt eben sind und sich gegenüberliegen. Streuverluste werden damit vermieden und die Leuchtrichtung, beispielsweise auf die Bodenplatte, wird mit den damit verbundenen positiven Wirkungen beibehalten.

Vorteilhafterweise sind der Lichtaustrittsabschnitt und der Lichteintrittsabschnitt um einen vorbestimmten Winkel bezogen auf die Ebene der Bodenplatte geneigt, bevorzugt um einen Winkel von ca. 20° bis ca. 70°. Dadurch ergibt sich eine besonders gute Ausleuchtung des Flüssigkeitsbehälters. Erfindungsgemäß ist der Lichtaustrittsabschnitt als ein transparenter Wandabschnitt des Gehäuses ausgebildet. In einer nicht beanspruchten Ausführungsform ist der Lichtaustrittsabschnitt als ein Fenster oder als eine Öffnung im Gehäuse ausgebildet. Je stärker die Transparenz, desto besser ist die optische Ausbeute für die Beleuchtung des Flüssigkeitsbehälters. Der transparente Wandabschnitt und das Fenster haben im Gegensatz zu einer Öffnung im Gehäuse den Vorteil, dass die Bauelemente im Gehäuseinneren vollständig umschlossen und damit geschützt sind.

Bevorzugt ist die Außenfläche des Lichteintrittsabschnitts zumindest abschnittsweise poliert. Dadurch wird eine Lichtstreuung vermieden und so die Lichtausbeute optimiert. In ähnlicher Weise ist es vorteilhaft, wenn die Innenfläche des Lichteintrittsabschnitts zumindest mattiert oder strukturiert ist. Damit wird hier eine möglichst große Streuung eines gegebenenfalls kleinen Lichtstrahls innerhalb des Flüssigkeitsbehälters erzielt.

Mit weiterem Vorteil weist das Leuchtmittel bzw. die Lichtquelle eine oder mehrere Leuchtdioden (LEDs) auf. Alternativ können jedoch auch andere Lichtquellen wie Halogenlampen, Glühbirnen, Laser oder andere geeignete Leuchtmittel verwendet werden. Insbesondere ist es vorteilhaft, wenn das Leuchtmittel mehrfarbig ist, z. B. eine mehrfarbige LED-Anordnung. Dies bietet die Möglichkeit, verschiedene funktionale Alarme mit verschiedenen Farben zu kodieren.

Es ist auch vorteilhaft, dass die Bodenplatte auf der Innenfläche poliert ist. Damit wird einfallendes Licht besser reflektiert. Dies führt zu einer stärkeren Lichtausbeute bei einem Beleuchtungsereignis.

Weiterhin erfindungsgemäß ist ein Atemluftbefeuchter mit einem Gehäuse, einem Flüssigkeitsbehälter und einer Beleuchtungsvorrichtung wie oben definiert. Bevorzugt weist der Atemluftbefeuchter eine Steuereinrichtung auf, die geeignet ist, die Beleuchtungsvorrichtung zu aktivieren und zu deaktivieren. Da in der Steuereinrichtung die Messwerte von Sensoren oder Daten von anderen, über elektrische Schnittstellen angebundenen Geräten, ausgewertet werden, ist es sinnvoll, entsprechend den Mess- oder Datenwerten die Beleuchtungsvorrichtung zu aktivieren oder zu deaktivieren. Besonders vorteilhaft ist es, wenn die Über- oder Unterschreitung eines Messwerts der Steuereinrichtung die Aktivierung der Beleuchtungsvorrichtung auslöst. So können beispielsweise Alarme optisch deutlicher und für den Benutzer besser erkennbar dargestellt werden. Zusätzlich ist es möglich, neben den optischen Signalen auch akustische Alarmsignale auszugeben.

Mit weiterem Vorteil ist der Messwert ein Füllstandswert, ein Temperaturwert oder ein anderweitiges Beatmungssignal. Damit ist es auch möglich, beispielsweise ein Alarmsignal von einem beatmeten Patienten oder von dem Beatmungsgerät selbst auf dem Atemluftbefeuchter zur Anzeige zu bringen.

Das Leuchtmittel bzw. die Lichtquelle kann in geeigneter Weise auf der oder den Leiterplatte(n) der Steuereinrichtung angeordnet sein. Dadurch lassen sich Raumverluste vermeiden. Darüber hinaus sind Beleuchtungsschaltungen mit integrierten Leuchtmitteln wie LEDs als integrierte Bauelemente günstig verfügbar und einfach handhabbar.

Die Erfindung soll nachfolgend anhand eines bevorzugten Ausführungsbeispiels unter Bezugnahme auf die beigefügten Figuren erläutert werden, in denen:
- Fig. 1: eine perspektivische Ansicht eines Gehäuses eines Atemluftbefeuchters mit einer erfindungsgemäßen Beleuchtungsvorrichtung gemäß einer bevorzugten Ausführungsform zeigt; und
- Fig.2: eine perspektivische Ansicht eines Atemluftbefeuchters zeigt, der eine bevorzugte Ausführungsform der erfindungsgemäßen Beleuchtungsvorrichtung aufweist;
- Fig. 3: eine perspektivische Schnittansicht des Atemluftbefeuchters mit der bevorzugten Ausführungsform der Beleuchtungsvorrichtung zeigt;
- Fig. 4: eine perspektivische Ansicht eines Flüssigkeitsbehälters für einen Atemluftbefeuchter gemäß einer bevorzugten Ausführungsform der vorliegenden Erfindung zeigt; und
- Fig. 5: eine perspektivische Schnittansicht des in Fig. 4 dargestellten Flüssigkeitsbehälters von unten zeigt.

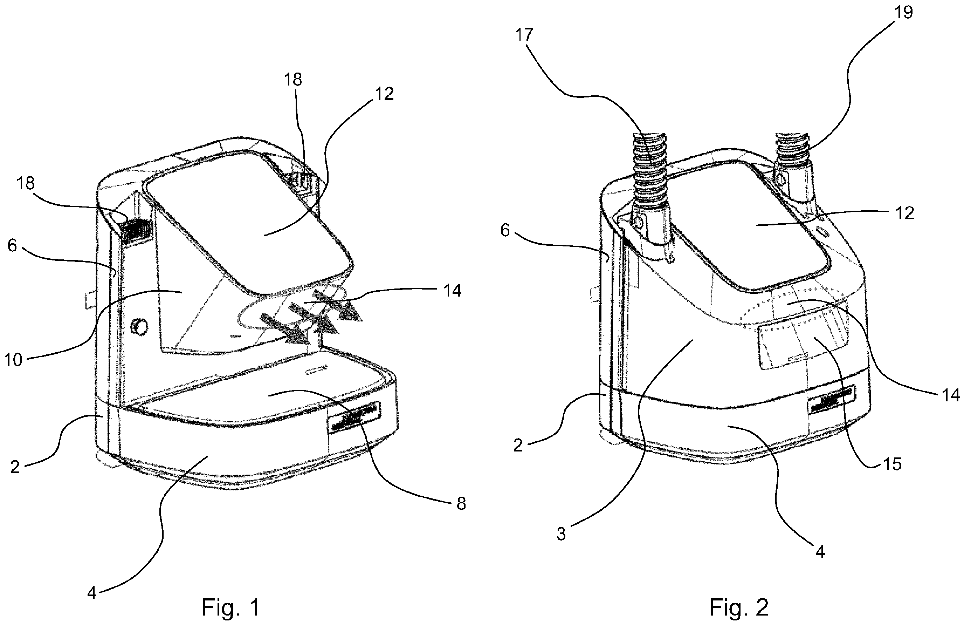

Fig. 1 zeigt in perspektivischer Ansicht das Gehäuse 2 eines Atemluftbefeuchters, der eine erfindungsgemäße Beleuchtungsvorrichtung gemäß einer bevorzugten Ausführungsform aufweist. Das Gehäuse 2 weist im Wesentlichen eine L-Form auf mit einem waagerechten Schenkel 4 und einem senkrechten Schenkel 6. An dem waagerechten Schenkel 4 ist eine Heizplatte 8 angeordnet, die im Wesentlichen waagerecht ausgerichtet ist und fast die gesamte Fläche des waagerechten Schenkels 4 bedeckt. Vom oberen Ende des senkrechten Schenkels 6 erstreckt sich etwa mittig ein vorsprungartiger Abschnitt 10, dessen schräge Oberfläche eine Benutzerschnittstelle 12 umfasst. Die Benutzerschnittstelle 12 weist (nicht dargestellte) Anzeigeeinrichtungen und Bedienelemente auf, mittels derer und einer (nicht dargestellten) Steuereinrichtung sich der Atemluftbefeuchter überwachen und steuern lässt. Seitlich des vorsprungartigen Abschnitts 10 sind am oberen Ende des senkrechten Schenkels 6 jeweils elektrische Kontaktelemente 18 angeordnet, die mit entsprechenden Verbindungsstücken eines Beamtungsschlauchsystems in elektrischen Kontakt gebracht werden können. Im in Richtung des waagerechten Schenkels 4 nach unten gerichteten Bereich des vorsprungartigen Abschnitts 10 weist das Gehäuse einen Abschnitt 14 auf, auf dessen Innenseite die Lichtquelle angeordnet ist und aus dem das Licht in Richtung des einzuschiebenden Flüssigkeitsbehälters austritt.

Fig. 2 zeigt in perspektivischer Darstellung einen Atemluftbefeuchter 1, der eine Beleuchtungsvorrichtung gemäß einer bevorzugten Ausführungsform aufweist. Wie in Verbindung mit Fig. 1 leicht erkennbar, ist auf das in Fig. 1 näher beschriebene Gehäuse 2 ein Flüssigkeitsbehälter 3 waagerecht aufgeschoben, der sich derart passend auf dem Gehäuse 2 anordnen lässt, so dass sich eine im Wesentlichen durchgängige Mantelfläche und eine im Wesentlichen durchgängige schräge Deckelfläche des Atemluftbefeuchters 1 ergibt. Seitlich aus dem oberen Abschnitt des Flüssigkeitsbehälters 3 ragen Atmungsschläuche 17 und 19 hervor, die über geeignete Verbindungssysteme mit dem Flüssigkeitsbehälter 3 und/oder den elektrischen Kontaktelementen 18 des Gehäuses 2 verbunden sind. Der vordere Abschnitt des Flüssigkeitsbehälters 3 weist einen Griff 15 auf, der zum Einschieben und Herausziehen des Flüssigkeitsbehälters 3 geeignet ist. Mit einer gepunkteten Linie ist der Abschnitt 14 gekennzeichnet, der angibt, dass in diesem Bereich Licht aus dem Inneren des Gehäuses 2 in den Flüssigkeitsbehälter 3 eintritt.

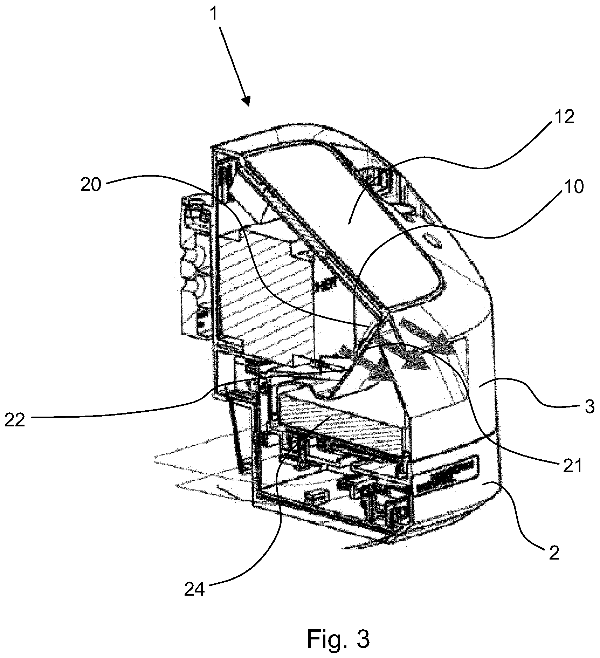

Fig.3 zeigt eine perspektivische Querschnittsansicht des Atemluftbefeuchters gemäß Fig. 2, der eine bevorzugte Ausführungsform der erfindungsgemäßen Beleuchtungsvorrichtung umfasst. Aus der Querschnittsansicht wird deutlich, dass im Inneren des vorsprungartigen Abschnitts 10 als Teil des Gehäuses 2 eine Lichtquelle bzw. ein Leuchtmittel 20 angeordnet ist. In dem schräg nach unten in Richtung der Bodenplatte des Flüssigkeitsbehälters 3 weisenden Abschnitts ist am Gehäuse 2 ein Lichtaustrittsabschnitt 22 ausgebildet, durch den das Licht in Richtung des Flüssigkeitsbehälters 3 hindurch tritt. Der Lichtaustrittsabschnitt 22 ist als im Vergleich zur umgebenden Wandstärke dünne Gehäusewand ausgebildet, die transparent ist. Dies hat den Vorteil, dass kein zusätzliches Bauelement mit einem anderen Material erforderlich ist. Alternativ kann der Lichtaustrittsabschnitt 22 auch als transparentes Glas- oder Kunststofffenster ausgebildet sein. Der Lichtaustrittsabschnitt 22 kann kreisförmig, ellipsenförmig, rechteckig oder polygonal geformt sein.

Parallel zu dem Lichtaustrittsabschnitt 22 ist in eng anliegender Konfiguration der Lichteintrittsabschnitt 21 des Flüssigkeitsbehälters 3 unmittelbar angrenzend angeordnet, durch den das Licht in den Flüssigkeitsbehälter 3 eintritt. Das Licht tritt in den Flüssigkeitsbehälter 3 ein und trifft dort auf die Oberfläche der Flüssigkeit 24 und wird dort gebrochen bzw. gestreut. Da der Flüssigkeitsbehälter 3 in der bevorzugten Ausführungsform aus transparentem Kunststoffmaterial gebildet ist, wird durch die Streuung der Reflexion des Lichts an den Innenflächen der Flüssigkeitsoberfläche das Licht über einen Großteil des Innenraums des Flüssigkeitsbehälters 3 verteilt und ist damit von außen sichtbar, und zwar nicht nur von vorne, das heißt entgegengesetzt der Leuchtrichtung, sondern auch in den seitlichen Abschnitten des Flüssigkeitsbehälters 3.

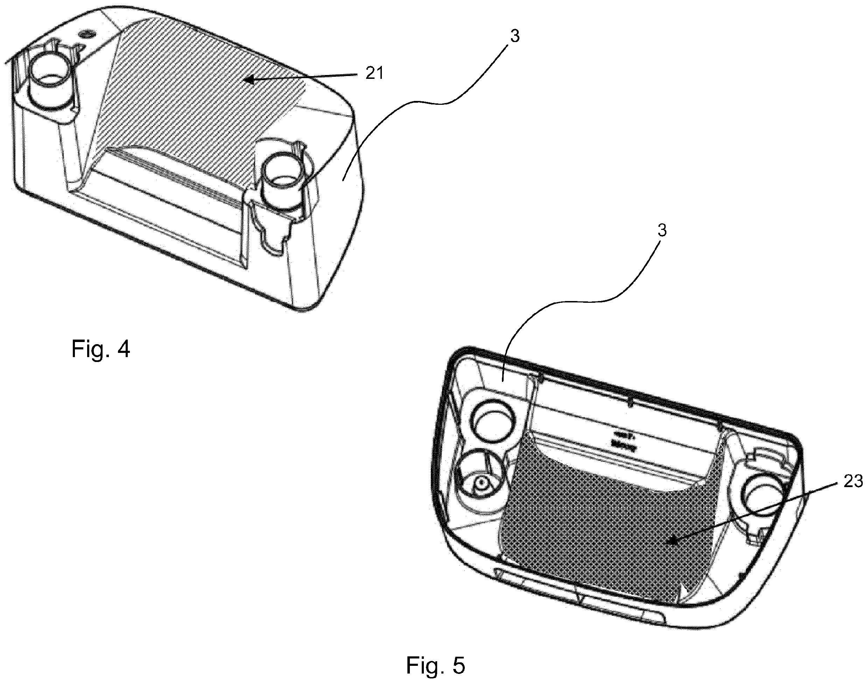

Fig. 4 zeigt in perspektivischer Darstellung den Flüssigkeitsbehälter 3 im Wesentlichen von oben, so dass der Lichteintrittsabschnitt 21 von oben sichtbar ist. Die Außenfläche des Lichteintrittsabschnitts ist zumindest abschnittsweise poliert. Die polierte Oberfläche soll bewirken, dass an dieser Fläche möglichst wenig Licht gestreut wird und folglich möglichst viel Licht direkt in den Flüssigkeitsbehälter 3 eindringt. Alternativ ist vorstellbar, dass der Lichteintrittsabschnitt 21 mit einer Folie beklebt ist oder einer Farbe entsprechend lackiert ist, so dass derselbe Effekt eintritt. Je größer der Lichteintrittsabschnitt 21, desto mehr Licht fällt in den Flüssigkeitsbehälter 3 ein, und dies erhöht wiederum die Signalwirkung der erfindungsgemäßen Beleuchtungsvorrichtung.

In Fig. 5 ist in einer perspektivischen Schnittansicht die Innenfläche 23 des Lichteintrittsabschnitts des Flüssigkeitsbehälters 3 dargestellt. Im Gegensatz zur Außenfläche 21 weist die Innenfläche 23 des Lichteintrittsabschnitts eine mattierte oder strukturierte Oberfläche auf. Damit wird bewirkt, dass das Licht innerhalb des Flüssigkeitsbehälters 3 möglichst breit gestreut wird. Auch hier ist es möglich, die erzielte Wirkung mit anderen Mitteln, wie zum Beispiel eine aufgeklebte Folie oder mittels eines aufgesprühten Materials, zu erreichen.

Insgesamt ergibt sich in der bevorzugten Ausführungsform der vorliegenden Erfindung, die in den Figuren dargestellt ist, eine sehr gute optische Signalquelle für Alarme, die von mehreren Seiten des Atemluftbefeuchters her sichtbar ist. Die Beleuchtungsvorrichtung macht sich dabei zu Nutze, dass auch das Wasser beleuchtet wird, das das Licht reflektiert und so zur Beleuchtung des gesamten Flüssigkeitsbehälters 3 von innen beiträgt. Auch die Bodenplatte des Flüssigkeitsbehälters kann gegebenenfalls besonders zur Lichtreflexion ausgebildet sein, z.B. poliert, wodurch die Lichtwirkung noch weiter verstärkt wird.

Mit dem Gegenstand der vorliegenden Erfindung wurde eine Beleuchtungsvorrichtung für einen Atemluftbefeuchter bereitgestellt, die einen Alarmzustand optisch sehr deutlich in möglichst viele Richtungen signalisieren kann und damit mit einem einfachen Aufbau kostengünstig realisierbar ist.

## Patentansprüche

1. Atemluftbefeuchter (1) mit Beleuchtungsvorrichtung, der einen Flüssigkeitsbehälter (3) mit einer Bodenplatte und ein Gehäuse (2) mit einer Heizplatte (8) aufweist, wobei zur Beheizung der Flüssigkeit im Flüssigkeitsbehälter (3) die Heizplatte (8) erwärmbar und die Bodenplatte in Kontakt mit der Heizplatte (8) bringbar ist, wobei das Gehäuse (2) ein Leuchtmittel (20) und einen Lichtaustrittsabschnitt (22) aufweist, und der Flüssigkeitsbehälter (3) einen Lichteintrittsabschnitt (21) aufweist, wobei das Licht ausgehend vom Leuchtmittel (20) in einer Leuchtrichtung durch den Lichtaustrittsabschnitt (22) und den Lichteintrittsabschnitt (21) in den Flüssigkeitsbehälter (3) eintritt, um einen Alarmzustand des Atemluftbefeuchters (1) optisch für einen Benutzer zu signalisieren,
**dadurch gekennzeichnet, dass**
der Lichtaustrittsabschnitt (22) als ein transparenter Wandabschnitt des Gehäuses (2) ausgebildet ist,
die Leuchtrichtung im Wesentlichen auf die Bodenplatte gerichtet ist,
und dass die Wandflächen des Flüssigkeitsbehälters (3) zumindest abschnittsweise transparent sind.

2. Atemluftbefeuchter (1)nach Anspruch 1, **dadurch gekennzeichnet, dass** der Lichtaustrittsabschnitt (22) und der Lichteintrittsabschnitt (21) zueinander passend geformte Oberflächen aufweisen, die mit einem Luftspalt dazwischen aneinander angrenzen.

3. Atemluftbefeuchter (1)nach einem der vorhergehenden Ansprüche, **dadurch gekennzeichnet, dass** der Lichtaustrittsabschnitt (22) und der Lichteintrittsabschnitt (21) eben sind und sich gegenüber liegen.

4. Atemluftbefeuchter (1)nach Anspruch 3, **dadurch gekennzeichnet, dass** der Lichtaustrittsabschnitt (22) und der Lichteintrittsabschnitt (21) um einen vorbestimmten Winkel bezogen auf die Ebene der Bodenplatte geneigt sind, der bevorzugt ca. 20° bis ca. 70° beträgt.

5. Atemluftbefeuchter (1)nach einem der vorhergehenden Ansprüche, **dadurch gekennzeichnet, dass** die Außenfläche des Lichteintrittsabschnitts (21) zumindest abschnittsweise poliert ist.

6. Atemluftbefeuchter (1)nach einem der vorhergehenden Ansprüche, **dadurch gekennzeichnet, dass** die Innenfläche (23) des Lichteintrittsabschnitts (21) zumindest abschnittsweise mattiert oder strukturiert ist.

7. Atemluftbefeuchter (1)nach einem der vorhergehenden Ansprüche, **dadurch gekennzeichnet, dass** das Leuchtmittel (20) eine oder mehrere Leuchtdioden (LEDs) aufweist.

8. Atemluftbefeuchter (1)nach einem der vorhergehenden Ansprüche, **dadurch gekennzeichnet, dass** das Leuchtmittel (20) mehrfarbig ist.

9. Atemluftbefeuchter (1)nach einem der vorhergehenden Ansprüche, **dadurch gekennzeichnet, dass** die Bodenplatte auf der Innenfläche poliert ist.

10. Atemluftbefeuchter (1) nach einem der vorhergehenden Ansprüche, **dadurch gekennzeichnet, dass** er eine Steuereinrichtung aufweist, die geeignet ist, die Beleuchtungsvorrichtung zu aktivieren und zu deaktivieren.

11. Atemluftbefeuchter (1) nach Anspruch 10, **dadurch gekennzeichnet, dass** die Über- oder Unterschreitung eines Messwertes der Steuereinrichtung die Aktivierung der Beleuchtungsvorrichtung auslöst.

12. Atemluftbefeuchter (1) nach Anspruch 11, **dadurch gekennzeichnet, dass** der Messwert ein Füllstandswert oder ein Temperaturwert ist.

## Claims

1. Respiratory humidifier (1) with lighting device, which has a fluid container (3) with a base plate, and a housing (2) with a heating plate (8), wherein in order to heat the fluid in the fluid container (3) the heating plate (8) can be heated up and the base plate can be brought into contact with the heating plate (8), wherein the housing (2) has a lamp (20) and a light emission section (22), and the fluid container (3) has a light admission section (21), wherein the light emanating from the lamp (20) moves in an illuminating direction through the light emission section (22) and light admission section (21) into the fluid container (3) in order to optically signal to a user a state of alarm of the respiratory humidifier (1), **characterised in that** the light emission section (22) is configured as a transparent wall section of the housing (2), the illuminating direction is directed substantially to the base plate, and that the wall surfaces of the fluid container (3) are transparent at least in some sections.

2. Respiratory humidifier (1) according to claim 1 **characterised in that** the light emission section (22) and the light admission section (21) have surfaces shaped to match one another and adjoin one another with an air space inbetween.

3. Respiratory humidifier (1) according to one of the preceding claims **characterised in that** the light emission section (22) and the light admission section (21) are flat and lie opposite one another.

4. Respiratory humidifier (1) according to claim 3 **characterised in that** the light emission section (22) and the light admission section (21) are inclined relative to the plane of the base plate by a predetermined angle, which is preferably about 20° to 70°.

5. Respiratory humidifier (1) according to one of the preceding claims **characterised in that** the outside surface of the light admission section (21) is polished at least in some sections.

6. Respiratory humidifier (6) according to one of the preceding claims **characterised in that** the inside surface (23) of the light admission section (21) is matted or structured at least in some sections.

7. Respiratory humidifier (1) according to one of the preceding claims **characterised in that** the lamp (20) has one or more light diodes (LEDs).

8. Respiratory humidifier (1) according to one of the preceding claims **characterised in that** the lamp (20) is multi-coloured.

9. Respiratory humidifier (1) according to one of the preceding claims **characterised in that** the base plate is polished on the inside surface.

10. Respiratory humidifier (1) according to one of the preceding claims **characterised in that** it has a control device which is suitable for activating and deactivating the lighting device.

11. Respiratory humidifier (1) according to claim 10 **characterised in that** exceeding or falling short of a measured value of the control device triggers the activation of the lighting device.

12. Respiratory humidifier (1) according to claim 11 **characterised in that** the measured value is a filling level value or temperature value

## Revendications

1. Humidificateur d'air inhalé (1) à dispositif d'éclairage, lequel humidificateur comprend un réservoir à liquide (3) pourvu d'une plaque de fond et un boîtier (2) pourvu d'une plaque de chauffe (8), la plaque de chauffe (8) pouvant être chauffée pour chauffer le liquide présent dans le réservoir à liquide (3) et la plaque de fond pouvant être amenée en contact avec la plaque de chauffe (8), le boîtier (2) comprenant un moyen d'éclairage (20) et une section de sortie de lumière (22), et le réservoir à liquide (3) comprenant une section d'entrée de lumière (21), la lumière sortant du moyen d'éclairage (20) entrant dans le réservoir à liquide (3), dans une direction d'éclairage, par la section de sortie de lumière (22) et la section d'entrée de lumière (21), afin de signaler optiquement à un utilisateur un état d'alarme de l'humidificateur d'air inhalé (1), **caractérisé en ce que** :
- la section de sortie de lumière (22) est réalisée sous la forme d'une section de paroi transparente du boîtier (2),
- la direction d'éclairage est orientée sensiblement vers la plaque de fond,
et **en ce que** les surfaces de paroi du réservoir à liquide (3) sont au moins en partie transparentes.

2. Humidificateur d'air inhalé (1) selon la revendication 1, **caractérisé en ce que** la section de sortie d'air (22) et la section d'entrée d'air (21) présentent des surfaces formées de manière à s'adapter les unes aux autres, qui sont adjacentes les unes aux autres et présentent un entrefer entre elles.

3. Humidificateur d'air inhalé (1) selon l'une quelconque des revendications précédentes, **caractérisé en ce que** la section de sortie de lumière (22) et la section d'entrée de lumière (21) sont plates et se font face.

4. Humidificateur d'air inhalé (1) selon la revendication 3, **caractérisé en ce que** la section de sortie de lumière (22) et la section d'entrée de lumière (21) sont inclinées selon un angle prédéfini par rapport au plan de la plaque de fond, qui atteint de préférence d'environ 20° à environ 70°.

5. Humidificateur d'air inhalé (1) selon l'une quelconque des revendications précédentes, **caractérisé en ce que** la surface extérieure de la section d'entrée de lumière (21) est au moins en partie polie.

6. Humidificateur d'air inhalé (1) selon l'une quelconque des revendications précédentes, **caractérisé en ce que** la surface intérieure (23) de la section d'entrée de lumière (21) est au moins en partie dépolie ou structurée.

7. Humidificateur d'air inhalé (1) selon l'une quelconque des revendications précédentes, **caractérisé en ce que** le moyen d'éclairage (20) comprend une ou plusieurs diodes électroluminescentes (DEL).

8. Humidificateur d'air inhalé (1) selon l'une quelconque des revendications précédentes, **caractérisé en ce que** le moyen d'éclairage (20) est multicolore.

9. Humidificateur d'air inhalé (1) selon l'une quelconque des revendications précédentes, **caractérisé en ce que** la plaque de fond est polie sur la surface intérieure.

10. Humidificateur d'air inhalé (1) selon l'une quelconque des revendications précédentes, **caractérisé en ce qu'**il comprend un système de commande qui est adapté à activer et à désactiver le dispositif d'éclairage.

11. Humidificateur d'air inhalé (1) selon la revendication 10, **caractérisé en ce que**, lorsqu'une valeur de mesure du système de commande est dépassée ou n'est pas atteinte, l'activation du dispositif d'éclairage est déclenchée.

12. Humidificateur d'air inhalé (1) selon la revendication 11, **caractérisé en ce que** la valeur de mesure est une valeur de niveau ou une valeur de température.
